(19) **Europäisches Patentamt**

**European Patent Office**

**Office européen des brevets**

(11) **EP 0 957 963 B1**

(12) **FASCICULE DE BREVET EUROPEEN**

(45) Date de publication et mention
de la délivrance du brevet:
**17.09.2003 Bulletin 2003/38**

(51) Int Cl.⁷: **A61M 16/00**, A62B 18/04

(86) Numéro de dépôt international:
**PCT/FR96/00684**

(21) Numéro de dépôt: **96919871.2**

(22) Date de dépôt: **06.05.1996**

(87) Numéro de publication internationale:
**WO 96/035468 (14.11.1996 Gazette 1996/50)**

(54) **DISPOSITIF OSCILLATOIRE DE PRESSION POUR DECOLLER LE MUCUS**

DRUCKSCHWINGUNGSVORRICHTUNG ZUR SCHLEIMLÖSUNG

OSCILLATORY PRESSURE DEVICE FOR REMOVING THE MUCUS

(84) Etats contractants désignés:
**DE ES FR GB IT NL SE**

(30) Priorité: **12.05.1995 FR 9505657**

(43) Date de publication de la demande:
**24.11.1999 Bulletin 1999/47**

(73) Titulaires:
• **Benarrouch, Jacques**
**F-69100 Villeurbanne (FR)**
• **Sangouard, Patrick**
**94350 Villiers-sur-Marne (FR)**

(72) Inventeurs:
• **Benarrouch, Jacques**
**F-69100 Villeurbanne (FR)**
• **Sangouard, Patrick**
**94350 Villiers-sur-Marne (FR)**

(74) Mandataire: **Schmitz, Jean-Marie et al**
**Dennemeyer & Associates Sàrl**
**P.O. Box 1502**
**1015 Luxembourg (LU)**

(56) Documents cités:
EP-A- 0 371 858          EP-A- 0 507 707
DE-A- 4 029 680          FR-A- 2 639 236

## Description

La présente invention concerne un dispositif destiné à extraire le mucus des parois bronchiques et pulmonaires pour les personnes atteintes en particulier de mucoviscidose dans le but d'améliorer leur état d'une façon significative en limitant les contraintes de soins.

En effet, les poumons sont recouverts par un film liquide de quelques micromètres normalement très fluide et qui protège les cellules pulmonaires en les isolant d'un contact direct avec l'air des poumons.

Des cils vibratiles oscillent librement dans ce film liquide normalement très fluide.

Rappelons que le problème fondamental lié à cette maladie génétique mortelle est que toutes les sécrétions exocrines des différents organes du corps sont anormalement beaucoup trop visqueuses, l'origine de cette hyper viscosité chez les mucoviscidosiques est génétique.

C'est pourquoi les malades atteints de mucoviscidose ont de graves difficultés digestives, mais surtout respiratoires.

C'est ainsi que ce mucus baignant les poumons entiers -et très difficile à évacuer- obstrue progressivement les alvéoles pulmonaires rendant la respiration difficile voire impossible.

Signalons que ce mucus, en plus de son action mécanique d'obstruction des alvéoles et de gène pour les oscillations naturelles des cils vibratiles, constitue un foyer important de contamination microbienne.

L'objectif que se propose d'atteindre cette invention est l'évacuation provoquée et contrôlée de tout ce mucus en surplus par modification de ses propriétés rhéologiques.

Jusqu'à présent, le traitement médical concernant l'expectoration du mucus est principalement constitué d'une kinésithérapie respiratoire qui se propose de favoriser l'évacuation du mucus par une pression contrôlée sur le thorax du patient. Cette méthode est appelée clapping.

Le patient peut alors évacuer une petite partie de ce mucus par des crachats. Mais, d'une part les vibrations du thorax exercées par le médecin pratiquant le clapping sont rapidement amorties dans les poumons et d'autre part, l'efficacité d'une telle méthode est limitée -par la viscosité du mucus- aux régions pulmonaires où les bronches ont une section suffisamment large.

L'invention repose sur la propriété -jusqu'ici jamais prise en compte- de thixotropie que possède le mucus pulmonaire. Par thixotropie, on entend: "phénomène par lequel certains mélanges passent de l'état de gel à celui de liquide par une légère agitation" (Larousse-Grand Dictionnaire Encyclopédique). En l'espèce, le mucus pulmonaire est thixotrope dans la mesure où de l'état visqueux il passe à l'état fluide lorsque l'agitation du mucus pulmonaire est provoquée par la vibration de l'air contenu dans les poumons et les bronches.

Cette thixotropie se produit à des fréquences de quelques hertz selon l'état du malade.

Il s'agira donc de générer dans tout le volume des poumons des variations oscillatoires de pression, de fréquence et d'amplitude appropriées à l'état spécifique du mucus du malade pour obtenir le minimum de viscosité de ce mucus.

Pour que ces variations de pression concernent les poumons entiers, il suffit que ce soit l'air inspiré par le malade qui vibre.

Afin d'expulser le mucus au-dehors, il est préférable que le phénomène de thixotropie se produise pendant la phase d'expiration.

L'expiration doit donc être contrôlée en provoquant une expectoration.

Cette toux s'obtient simplement, comme il sera décrit plus loin, en superposant aux variations oscillatoires de pression du volume d'air à l'intérieur des poumons une légère dépression par rapport à la pression atmosphérique de ce même volume d'air.

Cette dépression doit être suffisamment faibie en amplitude et courte en durée pour ne pas induire de collapsus des parois bronchiques.

Ces variations oscillatoires de pression sont évidemment arrêtées lors de la phase inspiratoire afin de ne pas produire un mouvement alternatif du mucus qui redevient alors très visqueux rapidement.

Le dispositif de l'invention ci-dessous décrit, facilite le décrochement par fluidification et l'évacuation du mucus des parois bronchiques par l'effet de variations oscillatoires périodiques et provoquées de dépression par rapport à la pression atmosphérique, variations d'amplitude croissante mais ajustable pour s'adapter à la viscosité spécifique du mucus bronchique, ce que ne permettent pas les brevets DE -A- 40 29 680 et US -A- 5 116 088.

En effet, le dispositif du brevet DE -A- 40 29 680 se caractérise par l'interruption périodique du flux d'air expiré dans un embout buccal obturable, impliquant une amplitude des vibrations de pression de l'air ni ajustable ni croissante; et selon le brevet US -A- 5 116 088, des volumes d'air pulsés à des fréquences ajustables mais en légère surpression par rapport à la pression de l'air inspiré sont délivrés durant la phase inspiratoire de la respiration.

Le dispositif simple de l'invention représente la base fondamentale d'une technologie susceptible d'être optimisée dans ses applications y compris par des fonctions associées en synergie que nous choisissons de ne pas développer ici.

Ce dispositif doit répondre aux contraintes suivantes:

1) -produire dans l'ensemble des bronches et des poumons des variations oscillatoires de dépression de l'air des poumons durant l'expiration, variations brusques, périodiques, d'amplitude croissante et de fréquence adaptée pour provoquer le phénomène de thixotropie du mucus pulmonaire,

2) -mettre à profit la fluidité obtenue du mucus pour le faire cheminer vers l'extérieur des poumons et l'évacuer par l'expectoration provoquée par la dépression pré-définie.

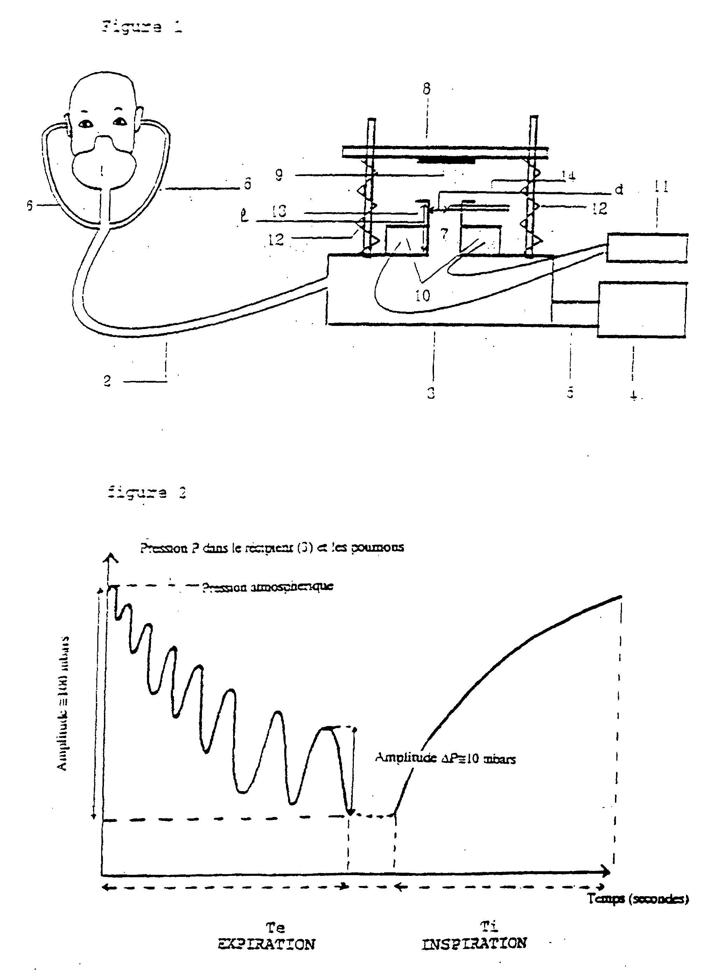

La figure 1 planche unique montre le principe fondamental du dispositif selon l'invention. Ce principe autorise la possibilité de construction d'un appareillage peu coûteux et peu encombrant ne. dépassant pas dix décimètres cubes.

Un masqué respiratoire (1) est apposé sur la bouche du malade. Ce masque est relié par un tuyau souple (2) à un récipient (3) dans lequel une pompe (4) crée une dépression d'environ 100 mbars réglable par une électrovanne (5) ayant une fuite variable avec l'atmosphère.

Deux autres petits conduits (6) équilibrent les pressions de part et d'autre du tympan pour éviter tout effet douloureux de résonance:

Un tube (7) de longueur ($\ell$) communiquant avec l'air ambiant par un orifice (13) de diamètre (d) variable grâce à un modulateur de débit (14) et faisant partie du récipient (3) est obturé hermétiquement et cycliquement par un clapet métallique (8) comportant une nappe élastique, de caoutchouc par exemple (9).

Cette obturation alternative est obtenue par une bobine (10) qui attire le clapet métallique (8) par un simple effet d'électroaimant. Lorsque aucun courant électrique ne circule dans la bobine (10) alimentée par le générateur basse fréquence (11), le clapet (8) est alors repoussé automatiquement par les ressorts (12).

Les variations oscillatoires de dépression ∆P obtenues dans le récipient et donc dans les poumons du malade suivent la loi suivante :

$$\Delta P = \frac{\pi.d^4.Pa.(Pa-P)}{128.\eta.V0.f.l}$$

avec :

d = diamètre de l'orifice (13) du tube (7)
$\ell$ = longueur du tube (7)
$\eta$ = viscosité de l'air
V0 = volume du récipient (3) et des poumons
Pa = valeur de la pression atmosphérique
P = valeur de la pression (statique) dans le récipient (3) et les poumons
f = fréquence d'obturation de l'orifice (13)

L'ajustement du diamètre (d) de l'orifice (13), qui modifie l'amplitude des variations oscillatoires de dépression ∆P, peut être obtenu simplement en limitant l'admission de l'air ambiant par une modification de la position du modulateur de débit (14).

Ces variations oscillatoires de dépression ∆P peuvent aussi être obtenues en modifiant la longueur ($\ell$) du tube (7).

La courbe de pression dans le récipient (3) et les poumons est alors celle de la figure 2 planche unique :

- La durée d'expiration forcée Te et la dépression progressive P doivent être inférieures à la constante de temps liée au collapsus des parois bronchiques.
- La dépression progressive P induit l'expectoration forcée.
- Les variations oscillatoires de dépression ∆P d'amplitude croissante décollent le mucus des parois bronchiques et lorsque la fréquence est amenée à sa valeur optimale, provoquent le phénomène de thixotropie du mucus en le rendant donc plus fluide. Ce mucus est alors évacué à l'extérteur par l'expectoration forcée de l'air des poumons générée par la dépression.
- L'inspiration s'effectue elle sans vibration de pression afin de parer à toute éventualité de retour du mucus qui redevient alors plus visqueux dans les bronches et les poumons.

**Revendications**

1. Dispositif améliorant l'extraction du mucus bronchique pendant la phase expiratoire, par modification de ses propriétés rhéologiques, **caractérisé par** l'association d'une dépression progressive par rapport à la pression atmosphérique créée par une pompe (4) dans un récipient (3) et transmise à l'ensemble du volume pulmonaire grâce à un tuyau souple (2) et un masque respiratoire (1), concommittante à des variations oscillatoires de cette dépression, variations brusques, cycliques, d'amplitude croissante et de fréquence ajustable à la fréquence de thixotropie du mucus pulmonaire, grâce à l'obturation périodique d'un tube (7) de longueur ($\ell$) et d'orifice (13) de diamètre (d) communicant avec l'atmosphère et le récipient (3).

2. Dispositif améliorant l'extraction du mucus bronchique pendànt la phase expiratoire, par modification de ses propriétés rhéologiques selon la revendication 1, **caractérisé en ce que** le récipient (3) avec le tube (7) devient un variateur oscillatoire de dépression ayant des amplitudes de vibrations progressivement croissantes par l'effet d'un clapet métallique (8), qui obture cycliquement l'orifice (13) du tube (7) reliant l'atmosphère avec le récipient (3) mis en dépression progressive par l'action d'une pompe (4), grâce à l'action d'une bobine (10) commandée par un générateur basse fréquence (11) et jouant le rôle d'un électro-aimant.

3. Dispositif améliorant l'extraction du mucus bronchique pendant la phase expiratoire, par modification de ses propriétés rhéologiques selon les revendications 1 et 2, **caractérisé en ce que** le variateur oscillatoire de dépression constitué par l'association d'une pompe (4), du récipient (3) et d'un orifice (13) obrurable sur l'atmosphère, puisse avoir des amplitudes de variations oscillatoires de dépression ∆P réglables grâce à une modification du débit d'air réalisé par la longueur (ℓ) variable du tube (7) ou le diamètre (d) de l'orifice (13) modifiable grâce à un modulateur de débit (14).

4. Dispositif améliorant l'extraction du mucus bronchique pendant la phase expiratoire, par modification de ses propriétés rhéologiques selon les revendications 1 et 2, **caractérisé par** la mise en place de deux conduits (6) mettant en communication les tympans avec l'air contenu dans les poumons et le tuyau (2).

**Patentansprüche**

1. Vorrichtung zur Verbesserung der Entfernung des Bronchialschleims während der Phase der Ausatmung durch Veränderung seiner rheologischen Eigenschaften, **gekennzeichnet durch** die Kombination eines bezüglich des atmosphärischen Drucks zunehmenden Unterdrucks, der **durch** eine Pumpe (4) in einem Behälter (3) erzeugt wird und über einen Schlauch (2) und eine Atemmaske (1) auf das gesamte Lungenvolumen übertragen wird, gleichzeitig mit schwingenden Änderungen dieses Unterdrucks, wobei diese Änderungen plötzlich, zyklisch, von steigender Amplitude und von einer Frequenz sind, die auf die Thixotropiefrequenz des Lungenschleims einstellbar ist, und zwar **durch** periodische Verschließung eines Rohrs (7) von der Länge (L) und mit einer Öffnung (13) mit dem Durchmesser (2), das mit der Atmosphäre und mit dem Behälter (3) in Verbindung ist.

2. Vorrichtung zur Verbesserung der Entfernung des Bronchialschleims während der Phase der Ausatmung durch Veränderung seiner rheologischen Eigenschaften nach Anspruch 1, **dadurch gekennzeichnet, dass** der Behälter (3) mit dem Rohr (7) ein schwingender Unterdruckvariator wird, dessen Schwingungsamplituden durch die Wirkung einer Metallklappe (8) allmählich zunehmen, die die Öffnung (13) des Rohrs (7) zyklisch verschließt, das die Atmosphäre mit dem Behälter (3) verbindet, der durch die Einwirkung einer Pumpe (4) unter zunehmenden Unterdruck gesetzt wird, und zwar durch die Einwirkung einer Spule (10), die durch einen Niederfrequenzgenerator (11) gesteuert wird und als Elektromagnet arbeitet.

3. Vorrichtung zur Verbesserung der Entfernung des Bronchialschleims während der Phase der Ausatmung durch Veränderung seiner rheologischen Eigenschaften nach den Ansprüchen 1 und 2, **dadurch gekennzeichnet, dass** der schwingende Unterdruckwandler, der durch die Kombination einer Pumpe (4), des Behälters (3) und einer verschließbaren Öffnung (13) zur Atmosphäre gebildet wird, Amplituden von schwingenden Unterdruckänderungen ∆P haben kann, die durch eine Änderung des Luftdurchsatzes regelbar sind, der durch die veränderliche Länge (L) des Rohrs (7) oder den Durchmesser (d) der Öffnung, der durch einen Durchsatzmodulator (14) veränderbar ist, erzeugt wird.

4. Vorrichtung zur Verbesserung der Entfernung des Bronchialschleims während der Phase der Ausatmung durch Veränderung seiner rheologischen Eigenschaften nach den Ansprüchen 1 und 2, **gekennzeichnet durch** die Anbringung von zwei Leitungen (6), die die Trommelfelle mit der in den Lungen und im Rohr (2) enthaltenen Luft in Verbindung setzen.

**Claims**

1. Device for improving the extraction of bronchial mucus during exhalation through modification of its rheological properties, **characterized by** the association of a gradual pressure drop compared to atmospheric pressure created by a pump (4) in a recipient (3) and transmitted to the entire pulmonary volume by means of a flexible tube (2) and a respiratory mask (1), together with oscillatory variations of this negative pressure, the variations being sudden, cyclical variations of increasing amplitude and frequency adjustable to the thixotropic frequency of pulmonary mucus, thanks to periodic closure of a tube (7) of length (1) and orifice (13) of diameter (d) communicating with the atmosphere and the recipient (3).

2. Device for improving the extraction of bronchial mucus during exhalation through modification of its rheological properties according to claim 1, **characterized in that** the recipient (3) with the tube (7) becomes an oscillatory negative pressure controller, the amplitude of whose vibrations increasing gradually through the effect of a metal check valve (8), which cyclically closes the orifice (13) of the tube (7) connecting the atmosphere with the recipient (3), which is subject to a gradual pressure drop through the action of a pump (4) by means of a coil (10), controlled by a low-frequency generator (11) and serving as an electromagnet.

3. Device for improving the extraction of bronchial mucus during exhalation through modification of its rheological properties according to claims 1 and 2, **characterized in that** the oscillatory negative pressure controller formed by the association of a pump (4), the recipient (3), and an orifice (13) which can be sealed to the atmosphere, may have oscillatory variations of negative pressure $\Delta P$ whose amplitudes can be adjusted through modification of the air flow realized by the variable length (1) of the tube (7) or the diameter (d) of the orifice (13) that can be modified by means of a flow control device (14).

4. Device for improving the extraction of bronchial mucus during exhalation through modification of its rheological properties according to claims 1 and 2 **characterized by** the use of two conduits (6) enabling communication between the tympana and air contained in the lungs and the tube (2).

Figure 1

figure 2